# EUROPEAN PATENT SPECIFICATION

(11) **EP 2 750 639 B1**
(45) Date of publication and mention of the grant of the patent: **12.10.2016**
(21) Application number: 12753391.7
(22) Date of filing: 28.08.2012
(51) Int. Cl.: A61F 5/445, A61F 5/449

(54) **OSTOMY BASE PLATE HAVING DISCREET PULLING TAB**
STOMABASISPLATTE MIT EINER DISKRETEN ZUGLASCHE
PLAQUE DE BASE POUR STOMIE AYANT UNE LANGUETTE DISCRÈTE

(30) Priority: 29.08.2011 DK 201170479
(43) Date of publication of application: 09.07.2014
(73) Proprietor: Coloplast A/S, 3050 Humblebæk (DK)
(72) Inventor: EDVARDSEN, Henrik, 2100 Copenhagen OE (DK); STROEBECH, Esben, DK-2970 Hoersholm (DK)
(86) International application number: PCT/DK2012/050315
(87) International publication number: WO 2013/029623

(56) References cited:
- WO-A1-2013/007262
- US-A- 5 312 381
- US-A1- 2009 299 309

## Description

### Technical field

The current invention relates to a base plate for use with an ostomy device, wherein the base plate has a pulling tab that is folded back so that it provides a discreet pulling tab with a reduced risk of getting caught in clothes.

### Background

In order to facilitate removal of ostomy devices after use pulling tabs, often referred to as 'ears', have been arranged at the outer edge of the base plates.

Known pulling tabs extend as an extension from the peripheral edge of the base plate and thus when the base plate is attached to the skin, the pulling tab is flush with the skin and thus often difficult to grip. Moreover, as it extends from the base plate as a single exposed part it is prone to get caught in clothes which tug the base plate during movement of the user's body, whereby there is a risk of detachment of the base plate.

In US 2009/299309 is disclosed an ostomy base plate with a pulling tab arranged on the outer edge of the base plate.

Thus, a need to improve well known pulling tabs exists.

### Brief description

The current invention relates to an ostomy base plate attachable to an ostomy collecting bag, wherein the ostomy base plate comprises a skin friendly adhesive for attaching the base plate to the surface of the skin, an inner edge defining a through going hole for receiving a stoma and a backing layer whereon the adhesive is disposed on a proximal side of said backing layer, the ostomy base plate further comprises a pulling tab arranged at an outer edge of the ostomy base plate, wherein the pulling tab forms an angle with the backing layer of less than 90° when no force is applied to the pulling tab.

The outer edge of the base plate is mainly defined by the adhesive layer which defines part of the base plate which can be attached to the skin. Thus, any part that extends there from will not be attached to the skin and can thus be used as a pulling tab provided that it extends far enough for a user to be able to grip.

In one embodiment, the pulling tab can be made flush with the backing layer, i.e. so that it extends mainly in the same or close to same plane as the backing layer.

By providing a pulling tab which extends back across the ostomy base plate instead of further out from the outer edge of the base plate it is possible to provide a base plate, and thereby also an ostomy device, which is more discreet and easy to get hold of. Moreover, the risk that the pulling tab is caught in clothes of the user is reduced. This further reduces the risk that the base plate is detached unintentionally.

Additional, by having a pulling tab as defined, any risk of creating pockets between the pulling tab and the skin when the base plate is worn is removed. Such pockets may collect moisture or water when the user is in the shower. Such pockets of water will increase the deterioration of the adhesive of the base plate. This can in turn create leakage as the water from the pockets penetrates between the skin and the base plate creating small channels for liquid to run in.

As defined by the claim, the pulling tab forms an angle with the backing layer of less than 90° degrees when no force is applied. This should be interpreted in such a way that the defined configuration can be found when the pulling tab is not in use, or before removal of the base plate, since during manipulation of the pulling tab it may be arranged in many different angles in respect to the base plate defined by how the user pulls the base plate off.

In the same way, prior art base plates with pulling tabs may also define angles of less than 90° when the user manipulates the pulling tab in order to remove the base plate. However, in these commonly known products the angle will be above 90° when the user is not handling the device and thus no force is applied to the pulling tab.

It should be understood that as defined herein the angle α is measured between the proximal surface of the backing layer, i.e. the surface facing away from the user when the base plate is worn, and the pulling tab.

The pulling tab can be formed from many different materials and have many different sizes and shapes as long as these are suitable to be grabbed between two fingers so as to facilitate removal of the base plate. Thus, in one embodiment the pulling tab can be formed of a sheet material. Advantageously, the pulling tab can be formed of the same material as the backing layer of the base plate and thus it is possible to form the pulling tab integrally with the backing layer.

The pulling tab can during production be folded backwards along a folding line.

The pulling tab may be in the form of a sheet or strip or string with one part attached to the backing layer and another part (the free end) not being attached to the backing layer. The non-attached part may, when no force is applied, in one embodiment overly the attached part, or it may define an angle of less than 90° degrees with the attached part. The attached part is substantially parallel with the backing layer.

The angle between the pulling tab and the backing layer is measured as the angle between the part of the pulling tab not being attached to the backing layer (the "free" end), when no force is applied, and the backing layer.

In another embodiment, the pulling tab is in the form of a string wherein the two end points of the string is attached to respective first and second attachment points on the outer edge of the base plate. The string can for example be welded or glued to the backing layer.

This forms a pulling hole, defined by the string and the outer edge between the two attachment points. By providing the pulling hole large enough for a finger to extend through a user can easily remove the base plate by using one finger only. This is particularly useful for users with reduced dexterity where the tweezers grip required to grip a regular pulling tab can be difficult to form and maintain.

In another embodiment the pull tab could be in the form of a string where only one end of the string is attached at the outer edge. The outer end forms a loop and is tied or otherwise attached to the middle of the string, thereby also creating a hole through a finger may be inserted in order to pull the base plate.

The pulling tab does not have to be attached on the edge exactly, but can be attached somewhere in the area around the outer edge. The main consideration to be done when attaching the pulling tab is that when the user pulls the tab, a peel is created when the base plate is pulled off, i.e. the base plate will lift the outer edge and peel the base plate of. Contrary, if the pulling tab is attached too far from the outer edge, a shear will be created when pulling the tab, wherein the force will be distributed over a large area of the base plate creating a very large opposing force that makes it practically impossible to remove the base plate without tearing the base plate or breaking the pulling tab.

In another aspect the invention relates to an ostomy device comprising a base plate as described above and an ostomy collecting bag.

The ostomy device can be formed as a one piece ostomy device, where the base plate and the ostomy bag are permanently attached to each other during manufacturing or as a two piece ostomy device where the user assembles the base plate and the ostomy pouch.

### Brief description of the drawings

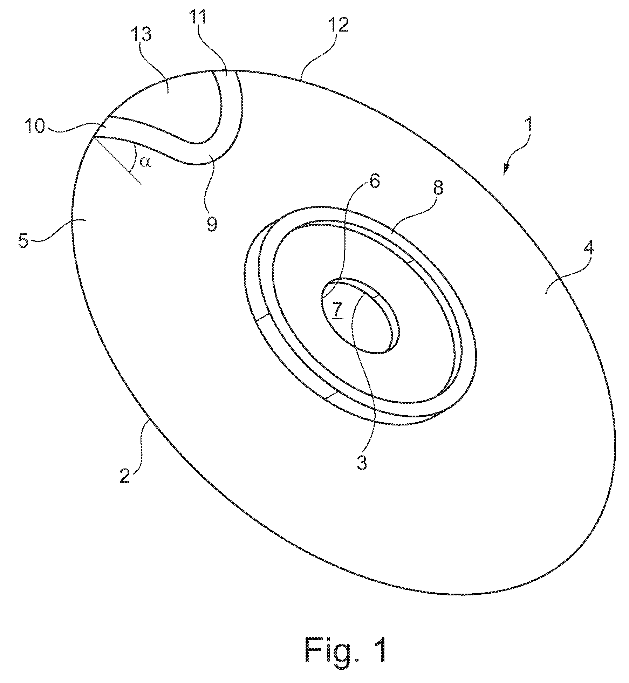

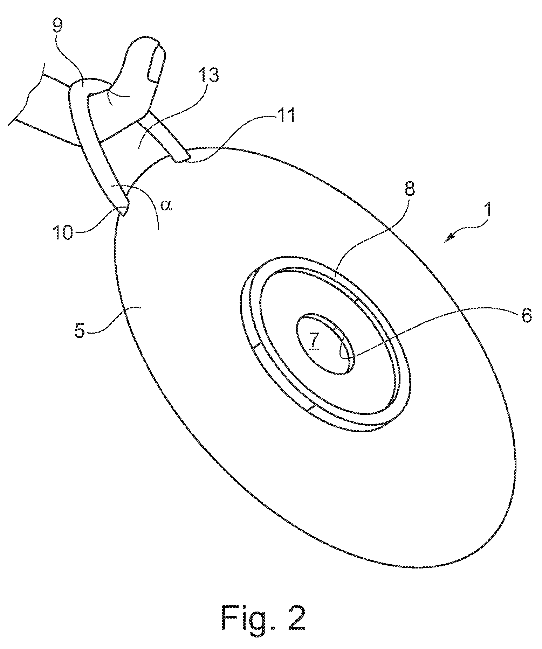

Fig. 1 and 2 show in perspective an embodiment of the invention.

### Detailed description

Figure 1 shows a base plate 1 having a proximal side 2, whereon an adhesive layer is disposed and a distal side 4 in the form of a backing layer 5. The base plate has an inner annular edge 6 which defines a through going hole 7.

On the distal side 4 there is provided a coupling ring 8, for coupling with an ostomy bag (not shown).

A pulling tab in the form of a string 9 having its two ends 10, 11 attached to an outer edge 12 of the base plate is provided.

The string and the outer edge defines a pulling hole 13 through which a finger can be inserted allowing a user to pull the tab with one finger.

The pulling tab extends backwards from the outer edge across the backing layer so that it forms an angle α with the backing layer of less than 90°. In the current example where the pulling tab is in the form of a string, the angle is measured between the backing layer and the surface of the string which faces the backing layer. The same way of measuring the angle can also be used where the pulling tab is a flap of sheet material or a part of the backing layer which has been folded back along itself along the outer edge of the base plate.

For example in figure 2 it can be seen how a user removes the base plate by inserting a finger through the pulling hole. During handling this may of course affect the angle α depending on how the user pulls off the base plate.

## Claims

1. An ostomy base plate (1) attachable to an ostomy collecting bag, wherein the ostomy base plate (1) comprises a skin friendly adhesive for attaching the base plate (1) to the surface of the skin, an inner edge defining a through going hole (7) for receiving a stoma and a backing layer (5) whereon the adhesive is disposed on a proximal side of said backing layer (5), the ostomy base plate (1) further comprises a pulling tab (9) arranged at outer edge (12) of the ostomy base plate, **characterized in that** the pulling tab (9) forms an angle with the backing layer (5) of less than 90º when no force is applied to the pulling tab (9).

2. An ostomy base plate according to claim 1, wherein the pulling tab is formed of sheet material.

3. An ostomy base plate according to claim 1 or 2, wherein the pulling tab is integrated with the backing layer.

4. An ostomy base plate according to claims 1, 2 or 3, wherein the pulling tab is in the form of a string, wherein the two end points of the string are attached to respective first and second attachment points on the outer edge of the base plate.

5. An ostomy base plate according to claims 1, 2, 3 or 4, wherein the pulling tab is flush with the backing layer.

6. An ostomy device comprising a base plate according to any one of the claims 1 - 5, and an ostomy collecting bag.

7. An ostomy device according to claim 6, wherein the ostomy device is a one piece ostomy device.

8. An ostomy device according to claim 6, wherein the ostomy device is a two piece ostomy device.

## Patentansprüche

1. Stoma-Basisplatte (1), die an einen Stoma-Sammelbeutel anbringbar ist, wobei die Stoma-Basisplatte (1) einen hautfreundlichen Klebstoff zum Anbringen der Basisplatte (1) an der Oberfläche der Haut, einen inneren Rand, der ein Durchgangsloch (7) zur Aufnahme eines Stomas definiert, und eine Trägerschicht (5), auf der der Klebstoff an einer proximalen Seite der Trägerschicht (5) angeordnet ist, umfasst, wobei die Stoma-Basisplatte (1) ferner eine Zuglasche (9) umfasst, die an dem äußeren Rand (12) der Stoma-Basisplatte angeordnet ist, **dadurch gekennzeichnet, dass** die Zuglasche (9) mit der Trägerschicht (5) einen Winkel von weniger als 90° bildet, wenn die Zuglasche (9) nicht mit Kraft beaufschlagt wird.

2. Stoma-Basisplatte nach Anspruch 1, wobei die Zuglasche aus Folienmaterial hergestellt ist.

3. Stoma-Basisplatte nach Anspruch 1 oder 2, wobei die Zuglasche mit der Trägerschicht integriert ist.

4. Stoma-Basisplatte nach Ansprüchen 1, 2 oder 3, wobei die Zuglasche in der Form eines Fadens ist, wobei die beiden Endpunkte des Fadens an einer ersten bzw. zweiten Anbringstelle am äußeren Rand der Basisplatte angebracht sind.

5. Stoma-Basisplatte nach Ansprüchen 1, 2, 3 oder 4, wobei die Zuglasche mit der Trägerschicht bündig ist.

6. Stoma-Vorrichtung, umfassend eine Basisplatte nach einem der Ansprüche 1 - 5 und einen Stoma-Sammelbeutel.

7. Stoma-Vorrichtung nach Anspruch 6, wobei die Stoma-Vorrichtung eine einteilige Stoma-Vorrichtung ist.

8. Stoma-Vorrichtung nach Anspruch 6, wobei die Stoma-Vorrichtung eine zweiteilige Stoma-Vorrichtung ist.

## Revendications

1. Plaque de base (1) pour stomie pouvant être fixée à une poche de collecte pour stomie, la plaque de base (1) pour stomie comprenant un adhésif compatible avec la peau servant à fixer la plaque de base (1) à la surface de la peau, un bord intérieur définissant un trou traversant (7) destiné à recevoir une stomie et une couche de support (5) sur laquelle est disposé l'adhésif sur un côté proximal de ladite couche de support (5), la plaque de base (1) pour stomie comprenant en outre une languette d'arrachement (9) disposée au niveau du bord extérieur (12) de la plaque de base pour stomie, **caractérisée en ce que** la languette d'arrachement (9) forme un angle avec la couche de support (5) inférieur à 90° lorsqu'aucune force n'est appliquée à la languette d'arrachement (9).

2. Plaque de base pour stomie selon la revendication 1, dans laquelle la languette d'arrachement est constituée d'un matériau en feuille.

3. Plaque de base pour stomie selon la revendication 1 ou 2, dans laquelle la languette d'arrachement est intégrée à la couche de support.

4. Plaque de base pour stomie selon la revendication 1, 2 ou 3, dans laquelle la languette d'arrachement se présente sous la forme d'un cordon, dans laquelle les deux points d'extrémité du cordon sont fixés à des premier et second points de fixation respectifs sur le bord extérieur de la plaque de base.

5. Plaque de base pour stomie selon la revendication 1, 2, 3 ou 4, dans laquelle la languette d'arrachement est au même niveau que la couche de support.

6. Appareillage pour stomie comprenant une plaque de base selon l'une quelconque des revendications 1 à 5 et une poche de collecte pour stomie.

7. Appareillage pour stomie selon la revendication 6, l'appareillage pour stomie étant un appareillage pour stomie d'un seul tenant.

8. Appareillage pour stomie selon la revendication 6, l'appareillage pour stomie étant un appareillage pour stomie en deux parties.
